# FASCICULE DE BREVET EUROPEEN

(11) **EP 0 707 005 B1**
(45) Date de publication et mention de la délivrance du brevet: **20.12.2000**
(21) Numéro de dépôt: 95402262.0
(22) Date de dépôt: 09.10.1995
(51) Int. Cl.: C07D 311/32, C07D 311/40, C07D 311/62

(54) **Procédé d'obtention d'oligomères procyanidoliques**
Verfahren zur Gewinnung von procyanidolischen Oligomeren
Process for obtaining of procyanidolic oligomerides

(30) Priorité: 11.10.1994 FR 9412126
(43) Date de publication de la demande: 17.04.1996
(73) Titulaire: SANOFI-SYNTHELABO, 75635 Paris Cédex 13 (FR)
(72) Inventeur: Franc, Bruno, F-30650 Saze (FR); Hoff, Christian, F-30126 St Laurent des Arbres (FR)
(74) Mandataire: Le Guen, Gérard

(56) Documents cités:
- CH-A- 626 359
- FR-A- 1 427 100
- FR-A- 2 092 743
- FR-A- 2 218 109
- FR-A- 2 372 823
- GB-A- 884 184

## Description

La présente invention se rapporte, d'une manière générale, à un procédé d'obtention des substances flavanoliques ou procyanidoliques présentes dans les végétaux.

Plus particulièrement, l'invention concerne une méthode d'extraction, à partir de végétaux, permettant d'obtenir à l'échelle industrielle, un extrait global des oligomères procyanidoliques qu'ils contiennent.

Ces oligomères procyanidoliques peuvent être définis comme représentant des mélanges relativement complexes de substances flavoniques, composées essentiellement de dérivés catéchiniques et de polymères de condensation de 2 à 5 molécules flavanoliques.

Ces mélanges constituent des substances homogènes qui présentent de remarquables propriétés pharmacologiques les rendant particulièrement utiles pour le traitement de maladies diverses particulièrement des affections du système circulatoire.

Par exemple, des oligomères procyanidoliques constitués d'un extrait de pépins de raisins purifié sont actuellement commercialisés pour leurs propriétés veino-protectrices.

Cet extrait répond à des normes standardisées à partir de spécifications analytiques précises définissant un équilibre entre monomères, oligomères et tanins.

On connaît déjà de nombreux procédés d'extraction des oligomères procyanidoliques, ou des substances similaires, présents dans les végétaux notamment dans le pépin de raisin qui en contient des quantités appréciables et qui de ce fait constitue une des sources potentielles les plus aptes à être utilisée industriellement.

On peut citer à cet effet, les procédés décrits dans les brevets FR 968 589, 1 036 922 et 1 427 100. Toutefois, aucun de ces procédés ne permet l'obtention globale des oligomères procyanidoliques présents dans les végétaux.

En outre, on peut citer le brevet, GB 884 184 qui rapporte un procédé permettant, lorsqu'il est appliqué à un végétal, de recueillir l'ensemble des oligomères procyanidoliques présents dans ce végétal.

Ce procédé est caractérisé en ce que le végétal choisi est extrait par de l'eau, à haute température en l'absence d'oxygène ou à basse température ou par des solvants organiques tels qu'un alcool ou de l'acétone, ou par un mélange de ceux-ci avec de l'eau, les impuretés également extraites étant précipitées en ajoutant, au milieu réactionnel, une solution aqueuse contenant 250 g / l de chlorure de sodium. Le filtrat est ensuite extrait par de l'acétate d'éthyle et le solvant est éliminé pour donner les oligomères procyanidoliques voulus.

Ce procédé, s'il permet une extraction totale des oligomères sans dénaturation de ceux-ci, ne peut être valablement utilisé à l'échelle industrielle d'abord en raison du faible rendement qu'il procure ensuite parce qu'il nécessite l'emploi d'une très grande quantité de chlorure de sodium soit 1 à 1,5 tonne par tonne de matières premières.

L'utilisation d'un tel procédé au niveau industriel provoquerait non seulement des difficultés de manutention et de stockage, mais également des problèmes d'élimination dus au chlorure de sodium.

De même; le brevet FR 2 372 823 décrit un procédé d'extraction des oligomères procyanidoliques reposant sur les étapes suivantes :
- extraction du végétal par un mélange acétone-eau contenant de 3 à 4 volumes d'acétone par volume d'eau,
- élimination de l'acétone par évaporation et filtration des impuretés,
- extraction du filtrat aqueux par l'acétate d'éthyle,
- élimination du solvant, reprise du résidu dans l'eau et sèchage sous vide,
- précipitation des impuretés par ajout d'une solution aqueuse saturée en chlorure de sodium et filtration,
- extraction du filtrat aqueux par l'acétate d'éthyle et évaporation partielle du solvant,
- précipitation des oligomères procyanidoliques par ajout de chloroforme puis sèchage.

Toutefois, ce procédé ne résout pas les inconvénients présentés par le procédé du brevet GB 884184 puisqu'il fait appel également à des solutions aqueuses chargées en chlorure de sodium qui, si elles sont abandonnées dans l'environnement, sont capables de provoquer de graves nuisances dues à la pollution.

Quant aux rendements offerts par ce procédé, ils s'avèrent extrêmement limités puisque de l'ordre de 0,6 %.

On mentionnera, au surplus, que les procédés antérieurs faisant appel à des mélanges eau/acétone à haute teneur en acétone provoquent l'extraction concomittante de résines qu'il est nécessaire de séparer dans une étape particulière.

La mise au point d'un procédé d'obtention des oligomères procyanidoliques apte à être utilisé sur le plan industriel et permettant de remédier aux inconvénients ci-dessus tout en fournissant un produit conforme aux normes standardisées et selon un rendement satisfaisant reste d'un intérêt majeur.

On a maintenant découvert qu'il est possible d'obtenir la totalité des oligomères procyanidoliques présents dans les végétaux selon un procédé industriel d'extraction capable de remédier aux inconvénients des procédés antérieurs tout en fournissant un rendement supérieur en oligomères analytiquement conformes.

Selon l'invention, on obtient les oligomères procyanidoliques par :
- traitement d'un végétal approprié au moyen d'un mélange extracteur à savoir un mélange composé d'eau, d'un solvant organique miscible à l'eau capable de dissoudre les oligomères procyanidoliques et de chlorure de sodium,
- purification du mélange brut d'oligomères procyanidoliques obtenu, par élimination du mélange extracteur et extraction du résidu,
- récupération des oligomères procyanidoliques par précipitation.

Selon une mise en oeuvre préférée du procédé ci-dessus, on recycle, dans la phase de traitement du végétal, le mélange extracteur après son élimination c'est-à-dire le solvant organique miscible à l'eau d'une part et la phase aqueuse chargée en chlorure de sodium d'autre part.

Le procédé de l'invention se caractérise, en conséquence, par une phase d'extraction du végétal réalisée par un mélange comportant 3 composants.

Cette phase, d'extraction est généralement, menée à chaud, c'est-à-dire à une température de l'ordre de 50 ° à 70 °C de préférence à une température de 55 ° à 60 °C pour obtenir, avec une cinétique acceptable, un épuisement correct du végétal par exemple des pépins de raisins.

A cet effet, on met en oeuvre un mélange extracteur constitué d'eau et d'un solvant organique miscible à l'eau capable de dissoudre les oligomères procyanidoliques, auquel on a ajouté du chlorure de sodium. Ce solvant organique miscible à l'eau, généralement un composé cétonique en C₁-C₄ tel que par exemple l'acétone, la méthyl éthyl cétone ou la méthyl isopropylcétone, est utilisé à raison de 25 à 50 % du poids de l'eau de préférence de 30 à 35 %.

Comme mélange extracteur on préfère habituellement un mélange eau-acétone-chlorure de sodium.

Quant au chlorure de sodium, on l'incorpore au mélange eau-solvant organique miscible à l'eau, à raison de 5 à 35 % du poids de l'eau selon la composition du mélange d'oligomères procyanidoliques à obtenir.

La concentration en chlorure de sodium joue en effet un rôle important dans la composition qualitative des oligomères procyanidoliques extraits.

On a remarqué, en effet, une forte variation de la répartition en monomères, oligomères et tanins en fonction de la concentration en sel. Par exemple, une concentration en chlorure de sodium de 360 g/l d'eau permet d'obtenir un extrait contenant 41 % en monomères mais dépourvu de tanins alors qu'une concentration de 180 g/l d'eau procure un extrait contenant 30 % de monomères avec un rapport oligomères/tanins mieux équilibré.

De manière à obtenir un extrait d'oligomères procyanidoliques conforme aux normes standards dont il est question précédemment, on utilise le chlorure de sodium de préférence à une concentration de 15 à 20 % du poids de l'eau par exemple à la concentration de 18 % du poids de l'eau.

Selon une mise en oeuvre particulière du procédé de l'invention, on obtient les oligomères procyanidoliques par application de la suite d'étapes ci-dessous :
1) traitement du végétal, par exemple des pépins de raisins, par un mélange extracteur composé d' eau, d'un solvant organique miscible à l'eau capable de dissoudre les oligomères procyanidoliques, de préférence l'acétone et de chlorure de sodium,
2) élimination du solvant organique miscible à l'eau par distillation et recyclage de ce distillat dans la phase de traitement du végétal,
3) extraction du résidu par un solvant organique non miscible à l'eau et capable de dissoudre les oligomères procyanidoliques présents dans ce résidu tel que par exemple l'acétate d'éthyle,
4) filtration de la suspension obtenue et extraction du filtrat par le solvant organique non miscible à l'eau utilisé à l'étape précédente, par exemple l'acétate d'éthyle, ou par tout autre solvant organique pouvant convenir à cet effet, pour obtenir un extractum organique et recyclage de la phase aqueuse chargée en chlorure de sodium dans la phase d'extraction du végétal,
5) concentration par évaporation partielle du solvant de l'extractum organique précédent, deshydratation et concentration par une nouvelle évaporation partielle du solvant,
6) précipitation et séchage des oligomères procyanidoliques, la précipitation étant effectuée en ajoutant, à la solution organique concentrée, un solvant organique qui ne dissout pas les oligomères tel que par exemple le dichlorométhane ou préférentiellement le dichloréthane.

Le procédé de l'invention présente, par rapport à l'art antérieur, des avantages indéniables que l'on peut résumer comme suit :
- élimination d'une étape de filtration d'impuretés (résines), située immédiatement après la phase d'extraction du végétal,
- mise en oeuvre d'un volume réduit de solvant organique miscible à l'eau tel que l'acétone
- remplacement du chloroforme par un agent organique nettement moins toxique tel que le dichloréthane. Outre cette caractéristique, le dichloréthane permet une meilleure élimination du solvant organique non miscible à l'eau par exemple l'acétate d'éthyle.

Au surplus, le procédé de l'invention dans son aspect préférentiel permet de recycler le composé organique miscible à l'eau tel que l'acétone et d'éviter le rejet de la phase aqueuse chargée en chlorure de sodium.

En outre, cette phase aqueuse salée peut être recyclée à plusieurs reprises sans répercussions défavorables sur la qualité des oligomères procyanidoliques obtenus.

Le procédé ainsi conçu permet d'obtenir les oligomères en question analytiquement conformes avec un rendement de 1,5 à 2,5 % c'est-à-dire 3 à 4 fois supérieur aux rendements offerts par l'état de la technique.

L'exemple, non limitatif suivant, illustre le procédé de l'invention.

### EXEMPLE

### Procédé continu d'obtention des oligomères procyanidoliques

On constitue d'abord un mélange extracteur de composition suivante :

| | |
|---|---|
| Eau désionisée | 1650 l |
| Chlorure de sodium | 300 kg |
| Acétone | 550 l |

A l'aide d'un extracteur continu à vis, on réalise alors une extraction de pépins de raisins finement broyés durant 2 heures à la température de 55 à 60 °C, à raison d'un débit de pépins de 20 kg/h et d'un débit de 100 l/h du mélange d'extraction circulant à contre-courant.

En sortie d'extracteur, avant clarification sur machine centrifuge, on refroidit le miscella à 20 °C à l'aide d'un échangeur, on le prive d'acétone par distillation sous pression réduite à température de 45 °C et on recycle le distillat tel quel dans la phase d'extraction des pépins de raisins.

On ajoute alors, au résidu obtenu, 1 kg de mousse de cellulose, comme adjuvant de filtration et 40 l d'acétate d'éthyle comme agent de floculation des résines. Ces quantités sont déterminées pour 5 heures de fonctionnement.

On filtre la suspension sur essoreuse rendue inerte par ajout d'une précouche de cellulose, on extrait le filtrat avec 5 fractions de 45 l d'acétate d'éthyle et on recycle la phase aqueuse épuisée dans la phase d'extraction des pépins de raisins après étêtage pour éliminer l'acétate d'éthyle entraîné.

On concentre l'extrait acétate d'éthyle sous pression réduite à température inférieure à 40 °C jusqu'à un volume résiduel de 125 l. On déshydrate le résidu obtenu par agitation avec 8 kg de sulfate de sodium, on filtre la suspension puis on concentre dans les mêmes conditions que précédemment jusqu'à un volume résiduel de 9 l.

Après refroidissement à 20 °C, on précipite alors les oligomères procyanidoliques par ajout de 45 l de dichloréthane.

Sous azote, on filtre alors sur filtre clos sècheur, le solide très fin obtenu et on le rince avec 6 l de dichloréthane. On obtient de cette manière de 1,5 kg à 2,5 kg d'oligomères procyanidoliques pour 100 kg de pépins de raisins (rendement : 1,5 à 2,5 %).

Le produit brut obtenu est ensuite débarrassé de ses solvants résiduels par dissolution dans l'eau puis atomisé.

## Revendications

1. Procédé d'obtention d'oligomères procyanidoliques caractérisé en ce que :
- l'on traite un végétal approprié au moyen d'un mélange extracteur composé d'eau, d'un solvant organique miscible à l'eau capable de dissoudre les oligomères procyanidoliques et de chlorure de sodium,
- on purifie le mélange brut d'oligomères procyanidoliques obtenu, par élimination du mélange extracteur et extraction du résidu,
- on récupère, par précipitation, les oligomères procyanidoliques sous forme pure.

2. Procédé selon la Revendication 1 caractérisé en ce que l'on recycle le mélange extracteur dans la phase de traitement du végétal.

3. Procédé selon la Revendication 1 ou 2 caractérisé en ce que :
- l'on traite un végétal approprié par un mélange extracteur composé d'eau, d'un solvant miscible à l'eau capable de dissoudre les oligomères procyanidoliques et de chlorure de sodium,
- on élimine le solvant organique miscible à l'eau par distillation et on recycle ce distillat dans la phase de traitement du végétal,
- on extrait le résidu par un solvant organique non miscible à l'eau et capable de dissoudre les oligomères procyanidoliques présents dans ce résidu,
- on filtre la suspension obtenue et on extrait le filtrat par le solvant organique non miscible à l'eau ou par tout autre solvant organique pouvant convenir à cet effet, pour obtenir un extractum organique et on recycle la phase aqueuse chargée en chlorure de sodium dans la phase de traitement du végétal,
- on concentre par évaporation partielle le solvant de l'extractum organique, déshydrate et concentre à nouveau par évaporation partielle du solvant,
- on précipite et sèche les oligomères procyanidoliques, la précipitation étant effectuée en ajoutant, à la solutation organique concentrée, un solvant organique qui ne dissout pas les oligomères procyanidoliques.

4. Procédé selon une des Revendications 1 à 3 caractérisé en ce que le végétal est constitué de pépins de raisins.

5. Procédé selon une des Revendications 1 à 4 caractérisé en ce que le traitement du végétal se déroule à une température de 50 °C à 70 °C.

6. Procédé selon une des Revendications 1 à 5 caractérisé en ce que le solvant organique miscible à l'eau est utilisé à raison de 25 à 50 % du poids de l'eau.

7. Procédé selon la Revendication 6 caractérisé en ce que le solvant organique miscible à l'eau est utilisé à raison de 30 à 35 % du poids de l'eau.

8. Procédé selon une des Revendications 1 à 7 caractérisé en ce que le solvant miscible à l'eau est un composé cétonique en C₁-C₄.

9. Procédé selon la Revendication'8 caractérisé en ce que le composé cétonique en C₁-C₄ est l'acétone.

10. Procédé selon une des Revendications 1 à 9 caractérisé en ce que le chlorure de sodium est utilisé à raison de 5 à 35 % du poids de l'eau.

11. Procédé selon la Revendication 10 caractérisé en ce que le chlorure de sodium est utilisé à raison de 15 à 20 % du poids de l'eau.

12. Procédé selon une des Revendications 3 à 11 caractérisé en ce que le solvant organique non miscible à l'eau est l'acétate d'éthyle.

13. Procédé selon une des Revendication 3 à 12 caractérisé en ce que la précipitation a lieu par ajout de dichlorométhane ou dichloréthane à la solution organique concentrée.

## Patentansprüche

1. Verfahren zur Herstellung von procyanidolischen Oligomeren, dadurch gekennzeichnet, daß man:
- eine geeignete Pflanze mit einer Extraktionsmischung behandelt, die aus Wasser, einem mit Wasser mischbaren organischen Lösungsmittel, welches die procyanidolischen Oligomere zu lösen vermag, und Natriumchlorid gebildet ist,
- die erhaltene rohe Mischung von procyanidolischen Oligomeren durch Entfernen der Extraktionsmischung und Extraktion des Rückstands reinigt,
- die procyanidolischen Oligomeren in reiner Form durch Ausfällung gewinnt.

2. Verfahren nach Anspruch 1, dadurch gekennzeichnet, daß man die Extraktionsmischung in die Phase der Behandlung der Pflanze recyclisiert.

3. Verfahren nach Anspruch 1 oder 2, dadurch gekennzeichnet, daß man:
- eine geeignete Pflanze mit einer Extraktionsmischung behandelt, die aus Wasser, einem mit Wasser mischbaren Lösungsmittel, welches die procyanidolischen Oligomeren zu lösen vermag, und Natriumchlorid gebildet ist,
- das mit Wasser mischbare organische Lösungsmittel durch Destillation entfernt und das Destillat in die Phase der Behandlung der Pflanze recyclisiert,
- den Rückstand mit einem organischen Lösungsmittel extrahiert, welches mit Wasser nicht mischbar ist und die in diesem Rückstand vorhandenen procyanidolischen Oligomeren zu lösen vermag,
- die erhaltene Suspension filtriert und das Filtrat mit dem mit Wasser nicht mischbaren Lösungsmittel oder irgendeinem hierzu geeigneten anderen organischen Lösungsmittel extrahiert zur Bildung eines organischen Extrakts, und die mit Natriumchlorid versetzte wäßrige Phase in die Phase der Behandlung der Pflanze recyclisiert,
- den organischen Extrakt durch teilweises Verdampfen des Lösungsmittels einengt, entwässert und erneut durch teilweises Verdampfen des Lösungsmittels einengt,
- die procyanidolischen Oligomeren ausfällt und trocknet, wobei die Ausfällung durch Zugabe eines organischen Lösungsmittels, welches die procyanidolischen Oligomeren nicht löst, zu der konzentrierten organischen Lösung bewirkt wird.

4. Verfahren nach einem der Ansprüche 1 bis 3, dadurch gekennzeichnet, daß die Pflanze durch Traubenkerne gebildet wird.

5. Verfahren nach einem der Ansprüche 1 bis 4, dadurch gekennzeichnet, daß die Behandlung der Pflanze bei einer Temperatur von 50°C bis 70°C erfolgt.

6. Verfahren nach einem der Ansprüche 1 bis 5, dadurch gekennzeichnet, daß das mit Wasser mischbare organische Lösungsmittel in einer Menge von 25 bis 50 %, bezogen auf das Gewicht des Wasser, verwendet wird.

7. Verfahren nach Anspruch 6, dadurch gekennzeichnet, daß das mit Wasser mischbare organische Lösungsmittel in einer Menge von 30 bis 35 %, bezogen auf das Gewicht des Wassers, eingesetzt wird.

8. Verfahren nach einem der Ansprüche 1 bis 7, dadurch gekennzeichnet, daß das mit Wasser mischbare Lösungsmittel eine C₁-C₄-Ketonverbindung ist.

9. Verfahren nach Anspruch 8, dadurch gekennzeichnet, daß die C₁-C₄-Ketonverbindung Aceton ist.

10. Verfahren nach einem der Ansprüche 1 bis 9, dadurch gekennzeichnet, daß das Natriumchlorid in einer Menge von 5 bis 35 %, bezogen auf das Gewicht des Wassers, eingesetzt wird.

11. Verfahren nach Anspruch 10, dadurch gekennzeichnet, daß das Natrium in einer Menge von 15 bis 20 %, bezogen auf das Gewicht des Wassers, eingesetzt wird.

12. Verfahren nach einem der Ansprüche 3 bis 11, dadurch gekennzeichnet, daß das mit Wasser nicht mischbare organische Lösungsmittel Ethylacetat ist.

13. Verfahren nach einem der Ansprüche 3 bis 12, dadurch gekennzeichnet, daß die Ausfällung durch Zugabe von Dichlormethan oder Dichlorethan zu der konzentrierten organischen Lösung erfolgt.

## Claims

1. Process for obtaining procyanidolic oligomers, characterised in that:
a suitable plant material is treated with an extraction mixture made up of water, a water-miscible organic solvent capable of dissolving the procyanidolic oligomers, and sodium chloride,
the crude mixture of procyanidolic oligomers obtained is purified by eliminating the extraction mixture and extracting the residue,
the procyanidolic oligomers are recovered in pure form by precipitation.

2. Process according to claim 1, characterised in that the extraction mixture is recycled into the plant treatment step.

3. Process according to claim 1 or 2, characterised in that:
a suitable plant material is treated with an extraction mixture made up of water, a water-miscible solvent capable of dissolving the procyanidolic oligomers, and sodium chloride,
the water-miscible organic solvent is eliminated by distillation and this distillate is recycled into the plant treatment step,
the residue is extracted using a water-immiscible organic solvent capable of dissolving the procyanidolic oligomers present in this residue,
the resulting suspension is filtered and the filtrate is extracted with the water-immiscible organic solvent or any other organic solvent which may be suitable for this purpose, in order to obtain an organic extract, and the aqueous phase charged with sodium chloride is recycled into the plant treatment step,
the solvent in the organic extract is concentrated by partial evaporation, then it is dehydrated and reconcentrated by partial evaporation of the solvent,
the procyanidolic oligomers are precipitated and dried, the precipitation being carried out by adding an organic solvent which does not dissolve the procyanidolic oligomers to the concentrated organic solution.

4. Process according to one of claims 1 to 3,
characterised in that the plant material consists of grape pips.

5. Process according to one of claims 1 to 4,
characterised in that the treatment of the plant material is carried out at a temperature of 50°C to 70°C.

6. Process according to one of claims 1 to 5,
characterised in that the water-miscible organic solvent is used in an amount of 25 to 50% by weight, based on the water.

7. Process according to claim 6, characterised in that the water-miscible organic solvent is used in an amount of 30 to 35% by weight, based on the water.

8. Process according to one of claims 1 to 7,
characterised in that the water-miscible solvent is a C₁₋₄ ketone compound.

9. Process according to claim 8, characterised in that the C₁₋₄ ketone compound is acetone.

10. Process according to one of claims 1 to 9,
characterised in that the sodium chloride is used in an amount of 5 to 35% by weight, based on the water.

11. Process according to claim 10, characterised in that the sodium chloride is used in an amount of 15 to 20% by weight, based on the water.

12. Process according to one of claims 3 to 11,
characterised in that the water-immiscible organic solvent is ethyl acetate.

13. Process according to one of claims 3 to 12,
characterised in that the precipitation is carried out by adding dichloromethane or dichloroethane to the concentrated organic solution.
